# EUROPEAN PATENT SPECIFICATION

(11) **EP 4 417 141 B1**
(45) Date of publication and mention of the grant of the patent: **07.10.2026**
(21) Application number: 24179860.2
(22) Date of filing: 23.02.2016
(51) Int. Cl.: A61M 25/01, A61B 1/008, A61B 1/01, A61B 8/12, A61B 17/00

(54) **SINGLE PIECE BENDING NECK FOR AN ARTICULATING ULTRASOUND PROBE**
BIEGEHALS FÜR EINE GELENKIGE ULTRASCHALLSONDE
COL DE PLIAGE POUR SONDE ULTRASONORE ARTICULÉE

(30) Priority: 02.03.2015 US 201562126752 P
(43) Date of publication of application: 21.08.2024
(62) Document number(s) of the earlier application(s) in accordance with Art. 76 EPC: 16709591.8 / 3 264 968
(73) Proprietor: Koninklijke Philips N.V., 5656 AE Eindhoven (NL)
(72) Inventor: CUSCUNA, Dino Francesco, Eindhoven (NL); JINKS, Kathryn, Eindhoven (NL)
(74) Representative: Philips Intellectual Property & Standards

(56) References cited:
- US-A1- 2010 287 755
- US-A1- 2012 116 163
- US-A1- 2014 180 009

## Description

### FIELD

This invention relates to ultrasonic imaging probes and, in particular, to bending necks for articulating ultrasound probes.

### BACKGROUND

US 2014/0180009 discloses a bending portion for an endoscope, which includes a bending piece set in which a plurality of bending pieces are continuously provided by forming a convex portion on a first bending piece and a concave portion on a second bending piece by cutting a rigid pipe. The convex portion and the concave portion form an engagement portion that pivotably couples adjacent bending pieces to each other.

US 2010/0287755A1 discloses a method for manufacturing a bendable tube for an endoscopic instrument. First a tube is separated into several tube sections ina manner such that adjacent tube sections engage into one another with a positive fit in the axis direction of the tube. After the separation of the tube into several tube sections, one envisages peripherally arranging means for providing a positive fit transversely to the axis direction of the tube, on the region of the tube sections, which are situated I engagement with one another.

Some ultrasound probes are designed for imaging from within the body, including catheter probes and transesophageal echocardiography (TEE) probes. In these probes the imaging transducer is located at the tip of the probe, which is generally designed to be articulated by the operator so as to obtain the desired view. The preferred way to articulate the probe tip, particularly in the case of TEE probes, is by means of a distal section of the catheter or gastroscope referred to as a bending neck. The bending neck is formed by a series of links which are pivotally connected to each other. This enables each link to move slightly with respect to its adjoining links and hence the entire section of links can be made to controllably articulate over a substantial angle of bending. Control of the articulation is done by cables extending through the probe and the bending neck which are wrapped about the shaft or pulley of a control knob or motor in the control unit at the proximal end of the probe. As the operator turns a knob or actuates a motor, a desired cable is pulled, which bends the articulating neck section of the probe. Generally, the pivot axis between links alternates by 90° from link to link so that some axes can bend in the 0°-180° directions while the others can bend in the 90°-270° directions. The use of two controls and control cables for these two axis directions enables the operator to articulate the bending neck in any of these directions or any direction in between. The links and hence the bending neck is hollow, enabling the wiring for the transducer at the distal tip as well as other items such as guide wires and surgical tools to pass through the probe for operation at or through the tip of the probe.

The fabrication and assembly of a bending neck for an articulating probe can be painstaking and costly. Each link of the neck must be individually formed, then the links are joined by pins or rivets so that they will pivot with respect to each other. It is desirable to have an easier and less costly way to build a bending neck, yet still have the wide range of articulation and articulation control which users demand.

### SUMMARY

The invention is defined by the independent claims. The dependent claims define advantageous embodiments.

In accordance with an example, a bending neck for a controllably articulating ultrasound probe is provided which is formed from a nested tube set. The tube is etched or machined to form individual, pivoting links. A groove formed in one of the tubes of the nested tube set provides the control cable passageway. The bending neck curvature is formed to be variable, as by the use of movable bending points, multiple control cable anchor points, varying pivot axis spacing, and multi-durometer neck sheaths.

### BRIEF DESCRIPTION OF THE DRAWINGS

In the drawings:
Figs. 1 and 1A illustrate a section of a bending neck formed from a single nested set of two tubes.
Figs. 2 and 2A illustrate a section of a bending neck formed from a single tube (outside the scope of the claims), including an integral control cable passageway.
Fig. 3 illustrates a bending neck of the present invention with a variable bending deflection point.
Fig. 4 illustrates a bending neck with varied link spacing to provide variable articulation.
Fig. 5 is a detailed view of a technique for determining the link-to-link articulation angle for a bending neck of the present invention.
Fig. 6 illustrates a variable durometer sheath which provides variable bending for a bending neck of the present invention.
Figs. 7 and 8 illustrate the use of multiple control cable anchor points to controllably vary the bending of a bending neck of the present invention.
Fig. 9 illustrates a bending neck which can be controllably bent in two different planes through the use of differently anchored control cables.
Figs. 10a and 10b illustrate a variably articulating bending neck which is controllable articulated by two bending sections in one plane.
Figs. 11a and 11b illustrate a variably articulating bending neck which is controllable articulated by two bending sections in two planes.

### DESCRIPTION OF EMBODIMENTS

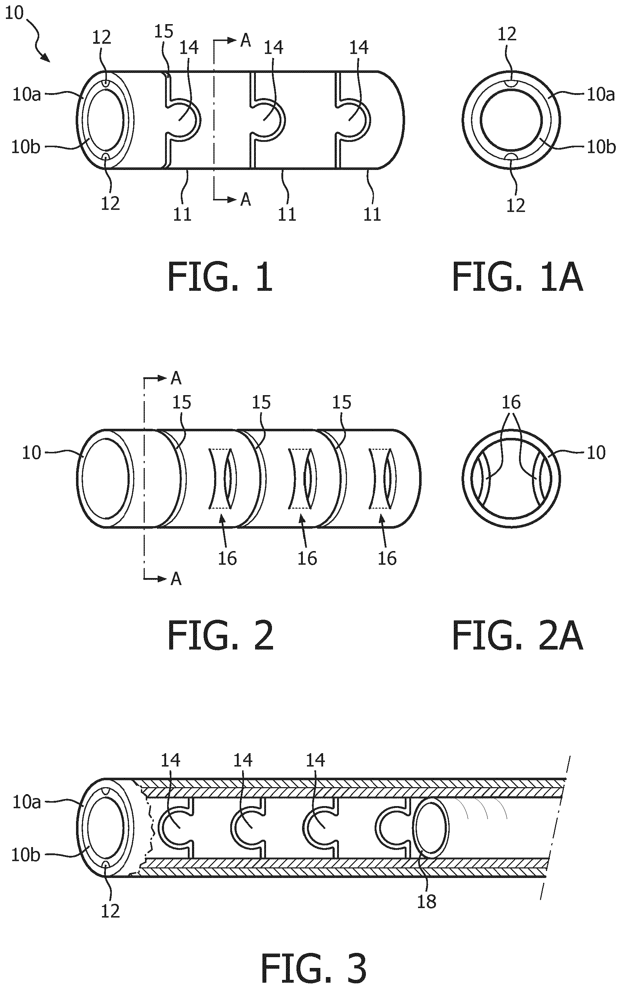

Referring first to Fig. 1, a single piece bending neck 10 for an articulating ultrasound probe is shown which is formed of two concentric tubes, generally made of a metal such as stainless steel. The inner tube 10b fits tightly within the outer tube 10a. Before insertion, two longitudinal grooves 12 are formed on opposite sides along the length of the outside of tube 10b. These grooves form passageways for control cables that control the articulation of the bending neck as described below. The grooves 12 are clearly shown in the cross-sectional view of Fig. 1A. With the two tubes concentrically positioned, they are then cut into separate links 11 by laser cutting toward the longitudinal axis of the tube or by another machining technique. The links are formed so as to remain movably connected to each other, as by lobes 14 extending from one link to the next and located on opposite sides of the links. These lobes and the spacing between the links formed by the machining process enable the adjacent links to move and pivot with respect to each other about axes extending through opposing lobes on opposite sides of the links. While each link may only pivot a small angle with respect to its neighbor, a number of successive links forming a bending neck may together bend in a considerable curve. This is the desired articulation, significant enough to be able to position the distal end of the probe where needed, but not sharp enough at any articular point so as to bind the wires, tools, and other items passing through the central lumen of the bending neck.

Fig. 2 illustrates a second implementation of a single piece bending neck, this time using just a single tube 10, which is outside the scope of the claims. The tube 10 is machined into separate connected links as described above, the grooves 15 between separate links being shown in this drawing. Since the inner tube used for the control cable groove is not present in this single tube implementation, a series of ring-like indentations 16 are formed on opposite sides of the tube to convey the control cables through the bending neck. Two parallel cuts are made through the tube wall, then the area between the cuts is pressed inward, forming the indentations as clearly shown in the cross-sectional view of Fig. 2A. The indentations are formed on the tube sides which are 90° around the tube from the lines of pivot lobes 14, which are on the top and bottom and cannot be seen in the view of Fig. 2. As the control cables passing though the indentations on opposite sides of the tube are pulled after being anchored at the distal end of the bending neck. They will respectively cause the neck to bend into and out of the plane of the drawing of Fig. 2.

There are a number of ways that the bending of a bending neck of the present invention can be controlled and adjusted. One control technique is to control the deflection point from which the bending takes place. Fig. 3 illustrates a technique in which a rigid member 18 is located in the bending neck with its distal end at the desired deflection point. In this case the rigid member is a tube 18 and this partially cut-away view shows links to the left of the tube 18 which are free to pivot about their pivot lobes, while the links through which the tube is located are immobilized from pivoting. The position of the deflection point is adjustable by adjusting the extension of the rigid member 18 into and out of the bending neck.

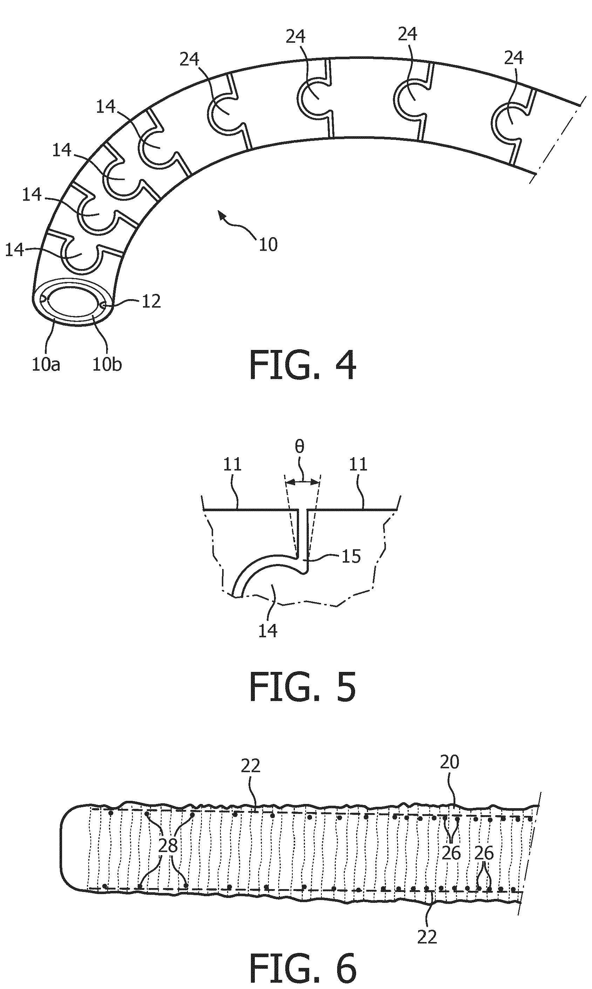

The angle subtended by the curvature of a section of the bending neck can be set by selectively determining the lengths of individual links as illustrated by the bending neck 10 of Fig. 4. In this implementation the links to the left with pivot lobes 14 are relatively short and the length of these links can bend with a relatively shorter radius of curvature. The larger links to the right with the pivot lobes 24 will bend maximally with a relatively larger radius of curvature. In addition, the different size links have different moments, which determine which set of links will bend first when commonly controlled. The smaller links with the pivot lobes 14, having smaller moments, will bend first. This is useful, for instance, when the placement of a transducer at the distal tip of the smaller links (left side of the drawing) is being controlled. The articulation of both sections of the bending neck is set to approximately the desired position by pulling relatively forcefully on the control cables in the grooves 12 and thereby causing both sections to bend. With the transducer near its desired position, light pulling of the cables is used to move only the distal section of smaller links to finely adjust the final desired position of the transducer.

The degree of pivoting between adjacent links is a function of the groove that is machined through the tube to form separate links. Fig. 5 is a partial side view of a portion of a bending neck where separate links 11 have been formed by machining groove 15 through the tube. The two links can pivot around pivot lobe 14 by the width of the groove 15, opening and closing the groove 90° on either side of the axis of the pivot lobes. If greater pivoting is desired the groove can be machined with a tapered width with a maximum opening of theta above and below the pivot lobes. The relative pivoting of the adjacent links is thereby increased to the dimension of angle theta.

Another technique for providing variable bending of a bending neck is to enclose the bending neck in a sheath with a variable durometer. Fig. 6 illustrates a sheath 20 over a bending neck with a variable durometer from the distal end to the left to the proximal end of the bending neck. The sheath is relatively stiffer (higher durometer) to the right, which becomes less stiff toward the distal end of the sheath. When the control cables are actuated to bend the bending neck, the distal end will bend first and more easily than the higher durometer proximal section of the bending neck. The durometer can be set by the choice of materials used along the length of the sheath. Another way to achieve the same result is to vary the thickness of the sheath material along the length of the sheath. The dashed lines 22 in Fig. 6 indicate that the sheath 20 is thicker toward its proximal (right) end than it is toward and at the distal end. Yet another way to achieve the same result is through the way in which the sheath is affixed to the bending neck. In the example of Fig. 6 the sheath 20 is tacked to the bending neck at closely spaced points 26 along the proximal portion of the bending neck, and is tacked to the bending neck at more widely spaced points 28 along the distal portion of the bending neck. This will cause the distal portion of the bending neck to bend more easily and readily than the proximal portion.

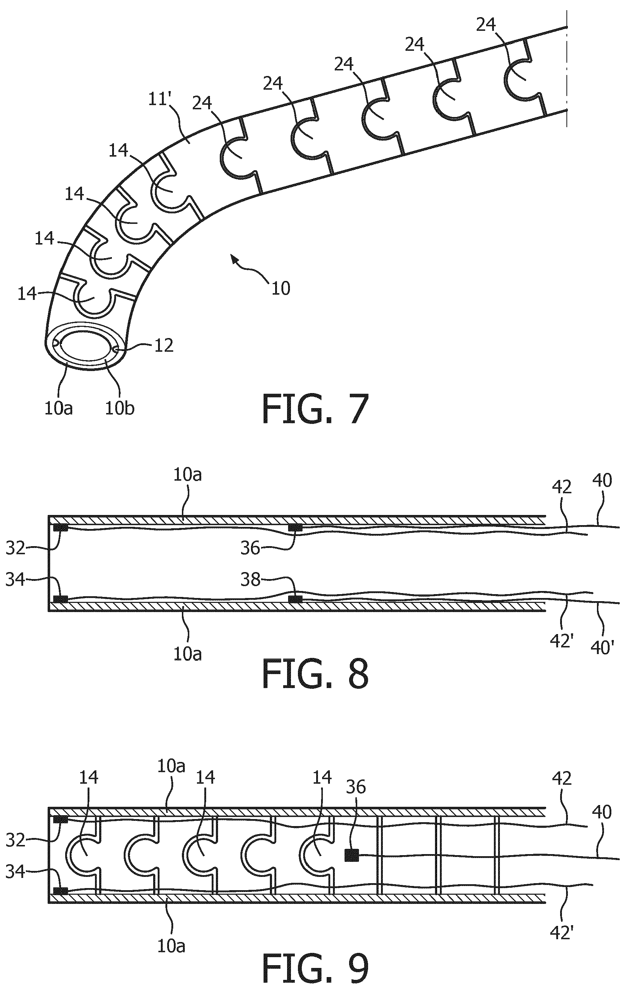

In some implementations it may be desirable to controllably bend a section of a bending neck at some times, and lock it in an unbent configuration at others. Fig. 7 illustrates an implementation of this feature using the embodiment of Fig. 4. In this case there are two sets of control cables, 40-42' and 42-42', extending through the control cable passageways 12. The ends of cables 40-40' are anchored by attachment to the distal link (leftmost) of the bending neck 10 as shown by anchor points 32 and 34 in Fig. 8. In Fig. 8 the inner tube 10b has been removed for clarity of illustration. The ends of the other set 42-42' of cables are anchored to link 11', the first link following those with pivot lobes 24, as shown by anchor points 36 and 38. When each pair of cables is pulled and relaxed in complementary fashion, the corresponding section of the bending neck is bent in the plane of the drawing, cable set 40-40' controlling the distal (small link) section and cable set 42-42' controlling the proximal (larger link) section. But when both of cables 42-42' are pulled in unison, the links of the proximal section are pulled together and locked into a straight configuration as shown in Fig. 7. The distal section of the bending neck can still be controllably articulated by use of cables 40-40'. When cables 40 and 40' are pulled in unison, the entire bending neck is locked in a straight configuration. Thus, by using multiple control cables and selected anchor points, different sections of a bending neck can be locked or articulated.

In the implementation of Fig. 7, the pivot lobes are all on the front and back of the bending neck, which allows both articulating sections to be curved in the same plane, the plane of the drawing. A single set of control cable passageways 12 accommodates both sets of cables for this articulation. Fig. 9 illustrates an implementation in which pivot lobes 14 are formed in the front and back sides of the tube and hence their pivot axes are all normal to the plane of the drawing. The pivot lobes 24 of the proximal section of the bending neck, however, are formed on the top and bottom of the tube and have their pivot axes parallel to the plane of the drawing. This means that the distal section with pivot lobes 14 can be curved in the plane of the drawing whereas the proximal section of links can be curved orthogonally into and out of the drawing plane. To control these different actions different sets of control cables are used. Cables 42 and 42' extend through cable passageways 12 and are anchored at the ends at anchor points 32 and 34. These cables control the articulation of the distal (leftmost) section of the bending neck. The control cables 40 and 40' for the proximal section of the bending neck are oriented 90° around the circumference of the tube from cables 42 and 42'. These control cables must pass through their own, differently positioned control cable passageways oriented 90° relative to passageways 12. These control cables 40 and 40' are anchored at the distal end of the section of links they control as shown by cable 40 anchored at anchor point 36 in the cutaway view of Fig. 9. (Cable 40' and its anchor point are cut away in this view.) When cables 42-42' are pulled the distal section of links is articulated or locked, and when cables 40-40' are pulled the proximal section of links is controlled.

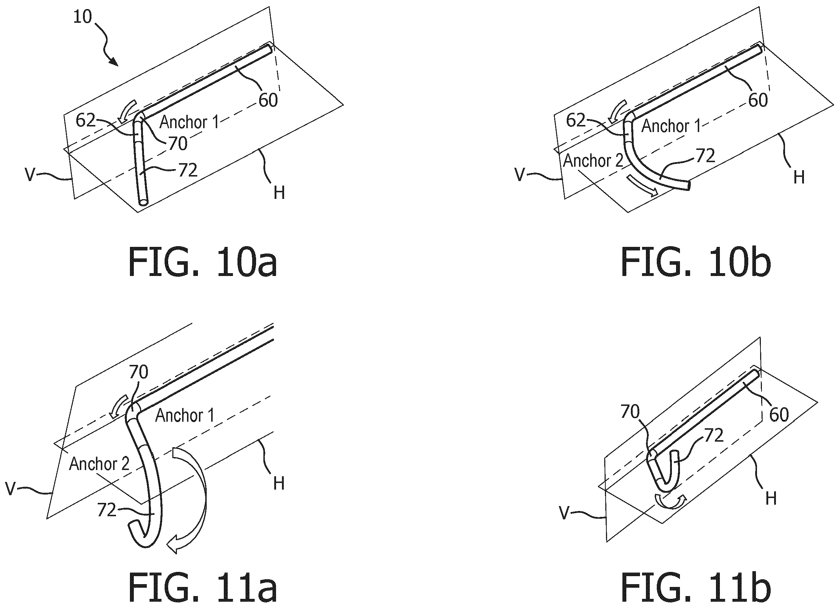

Figs. 10a-10b are perspective views of an articulating ultrasound probe of the present invention. This probe has two straight, non-articulating sections 60 and 62 and two articulating sections 70 and 72. Like the implementation of FIGURE 7, the articulating sections 70 and 72 articulate in the same plane, the horizontal plane H of the drawings. In Fig. 10a the short articulating section 70 is curved by control of its cables anchored at the distal end of section 70. In Fig. 10b the cable set anchored at the distal end of section 72 has been used to articulate section 72. Since all articulation is in the same plane, the pivot lobes of both sections are on the same sides of the section, and only a single pair of cable passageways is necessary for the control cables of both sections.

Figs. 11a-11b are perspective views of another articulating ultrasound probe of the present invention, this one implementing articulation in two planes as in the case of Fig. 9. Like Fig. 9, the articulating section 72 of Fig. 11a has its pivot lobes, pivot axes, and control cable passageways oriented 90° around the circumference of the tube as compared with those of articulating section 70. As Figs. 11a and 11b illustrate, the distal section 72 can be controllably articulated up and down in the vertical (V) direction.

Other variations of the above concepts will readily occur to those skilled in the art. The pivot lobes may be formed in other shapes and sizes, and pivoting between links can be provided by other, more complicated pin or rivet configurations. However, the implementations illustrated herein have the advantage of being wholly formed from a concentric pair of tubes. Instead of sections of identically oriented links, an articulating section can be interspersed with links pivoting at 90° with respect to each other, giving an articulating section the ability to be curved in almost any direction.

## Claims

1. An articulating neck (10) for an ultrasound probe comprising:
a plurality of separate and pivotally connected links (11), the links being formed from a first tube (10a) which has been machined with grooves (15) extending through the wall of the first tube by machining toward the longitudinal axis of the first tube, wherein each link comprises a pair of pivot lobes (14, 24) defined by the machined grooves (15) and located on opposite sides of the link, wherein the pivot lobes of each link extend into an adjoining link and enable the two links to pivot with respect to each other while remaining connected to each other; and **characterized by**
a second tube (10b), **characterised in that** said second tube is concentrically located inside the first tube, the second tube having been commonly machined with the first tube, and the second tube exhibiting first and second longitudinal grooves (12) extending longitudinally along the outer surface of the second tube, wherein the first and second longitudinal grooves (12) form passageways for control cables that control the articulation of the articulating neck.

2. The articulating neck of Claim 1, wherein the machining comprises laser cutting.

3. The articulating neck of Claim 1, wherein the pivot lobes (14, 24) of a link (11) further define a pivot axis extending through the pivot lobes.

4. The articulating neck of Claim 1, wherein the links of a section of consecutive links in the direction of the longitudinal axis exhibit the same length in the direction.

5. The articulating neck of Claim 4, further comprising a second section of consecutive links in the direction of the longitudinal axis,
wherein the links of the second section all exhibit the same length in the direction which is different than the length of the links of the first section.

6. The articulating neck of Claim 1,
wherein the pivot lobes of a plurality of successive links are aligned on opposite sides of the pivotally connected links, and
wherein the first and second longitudinal grooves (12) are located on opposite sides of the second tube (10b) and are circumferentially located at positions oriented 90° with respect to the aligned pivot lobes.

7. The articulating neck of Claim 1, wherein the pivot lobes of a plurality of successive links are aligned on opposite sides of the pivotally connected links, and
further comprising first and second indentations (16) extending into the central lumen of the link, the indentations forming ring-like passageways on opposite sides of the link through which a control cable may pass,
wherein the first and second indentations are located on opposite sides of the second tube and are circumferentially located at positions oriented 90° with respect to the aligned pivot lobes.

8. The articulating neck of Claim 1, wherein the machined grooves (15) further define pivot lobes on opposite sides of a link which pivotally connect one link to another, and
wherein the machined grooves exhibit a greater width 90° around the circumference of the link from the location of the pivot lobes.

9. An ultrasound probe comprising an articulating neck as claimed in any of the preceding claims.

## Patentansprüche

1. Gelenkiger Hals (10) für eine Ultraschallsonde, umfassend:
- eine Vielzahl separater und schwenkend verbundener Verbindungsglieder (11), wobei die Verbindungsglieder aus einem ersten Rohr (10a) gebildet sind, in das Nuten (15) eingearbeitet wurden, die sich durch die Wand des ersten Rohrs durch Bearbeiten in Richtung der Längsachse des ersten Rohrs erstrecken, wobei jedes Verbindungsglied ein Paar von Schwenklappen (14, 24) umfasst, die durch die eingearbeiteten Nuten (15) definiert sind und sich an entgegengesetzten Seiten des Verbindungsglieds befinden, wobei sich die Schwenklappen jedes Verbindungsglieds in ein angrenzendes Verbindungsglied erstrecken und es den beiden Verbindungsgliedern ermöglichen, zueinander zu schwenken, während sie miteinander verbunden bleiben; und **gekennzeichnet durch**
ein zweites Rohr (10b), **dadurch gekennzeichnet, dass** sich das zweite Rohr konzentrisch im Inneren des ersten Rohrs befindet, wobei das zweite Rohr gemeinsam mit dem ersten Rohr bearbeitet wurde, und wobei das zweite Rohr erste und zweite längliche Nuten (12) aufweist, die sich in Längsrichtung entlang der Außenfläche des zweiten Rohrs erstrecken, wobei die ersten und zweiten länglichen Nuten (12) Durchgänge für Steuerkabel bilden, die die Gelenkigkeit des gelenkigen Hals steuern.

2. Gelenkiger Hals nach Anspruch 1, wobei das Bearbeiten Laserschneiden umfasst.

3. Gelenkiger Hals nach Anspruch 1, wobei die Schwenklappen (14, 24) eines Verbindungsglieds (11) weiter eine Schwenkachse definieren, die sich durch die Schwenklappen erstreckt.

4. Gelenkiger Hals nach Anspruch 1, wobei die Verbindungsglieder eines Abschnitts aufeinanderfolgender Verbindungsglieder in der Richtung der Längsachse dieselbe Länge in der Richtung aufweisen.

5. Gelenkiger Hals nach Anspruch 4, weiter umfassend einen zweiten Abschnitt aufeinanderfolgender Verbindungsglieder in der Richtung der Längsachse,
wobei die Verbindungsglieder des zweiten Abschnitts dieselbe Länge in der Richtung aufweisen, die sich von der Länge der Verbindungsglieder des ersten Abschnitts unterscheidet.

6. Gelenkiger Hals nach Anspruch 1,
wobei die Schwenklappen einer Vielzahl aufeinanderfolgender Verbindungsglieder an entgegengesetzten Seiten der schwenkend verbundenen Verbindungsglieder ausgerichtet sind, und
wobei sich die ersten und die zweiten länglichen Nuten (12) an entgegengesetzten Seiten des zweiten Rohrs (10b) befinden und in Umfangsrichtung an Positionen liegen, die in einem Winkel von 90° zu den ausgerichteten Schwenklappen orientiert sind.

7. Gelenkiger Hals nach Anspruch 1, wobei die Schwenklappen einer Vielzahl aufeinanderfolgender Verbindungsglieder an entgegengesetzten Seiten der schwenkend verbundenen Verbindungsglieder ausgerichtet sind, und
weiter umfassend erste und zweite Einkerbungen (16), die sich in das zentralen Lumen des Verbindungsglieds erstrecken, wobei die Einkerbungen ringförmige Durchgänge an entgegengesetzten Seiten des Verbindungsglieds bilden, durch die ein Steuerkabel hindurchgehen kann,
wobei sich die ersten und zweiten Einkerbungen an entgegengesetzten Seiten des zweiten Rohrs befinden und in Umfangsrichtung an Positionen liegen, die in einem Winkel von 90° zu den ausgerichteten Schwenklappen orientiert sind.

8. Gelenkiger Hals nach Anspruch 1, wobei die bearbeiteten Nuten (15) weiter Schwenklappen an entgegengesetzten Seiten eines Verbindungsglieds definieren, die ein Verbindungsglied schwenkbar mit einem anderen verbinden, und
wobei die bearbeiteten Nuten eine größere Breite 90° um den Umfang des Verbindungsglieds von der Lage der Schwenklappen aus aufweisen.

9. Ultraschallsonde, umfassend einen gelenkigen Hals nach einem der vorstehenden Ansprüche.

## Revendications

1. Segment articulé (10) pour une sonde à ultrasons comprenant :
une pluralité de maillons séparés connectés de manière pivotante (11), les maillons étant formés à partir d'un premier tube (10a) qui a été usiné avec des rainures (15) s'étendant à travers la paroi du premier tube par usinage vers l'axe longitudinal du premier tube, dans lequel chaque maillon comprend une paire de lobes pivots (14, 24) définis par les rainures usinées (15) et situés sur des côtés opposés du maillon, dans lequel les lobes pivots de chaque maillon s'étendent dans un maillon adjacent et permettent aux deux maillons de pivoter l'un par rapport à l'autre tout en restant connectés l'un à l'autre ; et **caractérisé en ce que**
un second tube (10b), **caractérisé en ce que** ledit second tube est situé de manière concentrique à l'intérieur du premier tube, le second tube ayant été communément usiné avec le premier tube, et le second tube présentant des première et seconde rainures (12) s'étendant longitudinalement le long de la surface externe du second tube, dans lequel les première et seconde rainures longitudinales (12) forment des passages pour des câbles de commande qui commandent l'articulation du segment articulé.

2. Segment articulé selon la revendication 1, dans lequel l'usinage comprend en outre une découpe au laser.

3. Segment articulé selon la revendication 1, dans lequel les lobes pivots (14, 24) d'un maillon (11) définissent en outre un axe pivot s'étendant à travers les lobes pivots.

4. Segment articulé selon la revendication 1, dans lequel les maillons d'une section de maillons consécutifs dans la direction de l'axe longitudinal présentent la même longueur dans la direction.

5. Segment articulé selon la revendication 4, comprenant en outre une deuxième section de maillons consécutifs dans la direction de l'axe longitudinal,
dans lequel les maillons de la deuxième section présentent tous la même longueur dans la direction qui est différente de la longueur des maillons de la première section.

6. Segment articulé selon la revendication 1,
dans lequel les lobes pivots d'une pluralité de maillons successifs sont alignés sur les côtés opposés des maillons connectés de manière pivotante, et
dans lequel les première et seconde rainures (12) sont situées sur des côtés opposés du second tube (10b) et sont situées circonférentiellement dans des positions orientées à 90° par rapport aux lobes pivots alignés.

7. Segment articulé selon la revendication 1, dans lequel les lobes pivots d'une pluralité de maillons successifs sont alignés sur les côtés opposés des maillons connectés de manière pivotante, et
comprenant en outre des première et seconde indentations (16) s'étendant dans la lumière centrale du maillon, les indentations formant des passages annulaires sur les côtés opposés des maillons par lesquels passe un câble de commande,
dans lequel les première et seconde indentations sont situées sur des côtés opposés du second tube et sont situées circonférentiellement dans des positions orientées à 90° par rapport aux lobes pivots alignés.

8. Segment articulé selon la revendication 1, dans lequel les rainures usinées (15) définissent en outre des lobes pivots sur des côtés opposés d'un maillon qui connectent les maillons les uns aux autres de manière pivotante, et
dans lequel les rainures présentent une plus grande largeur à 90° autour de la circonférence du maillon à partir de l'emplacement des lobes pivots.

9. Sonde à ultrasons comprenant un segment articulé selon l'une quelconque des revendications précédentes.
